(19) Europäisches Patentamt
European Patent Office
Office européen des brevets

(11) Publication number: **0 067 593**
**A1**

(12) **EUROPEAN PATENT APPLICATION**

(21) Application number: 82302762.8

(22) Date of filing: 27.05.82

(51) Int. Cl.³: **C 07 D 239/48**
C 07 C 121/43, C 07 C 121/75

(30) Priority: 28.05.81 FI 811638

(43) Date of publication of application: 22.12.82 Bulletin 82/51

(84) Designated Contracting States: AT BE CH DE FR GB IT LI NL SE

(71) Applicant: Farmos-Yhtymä Oy
P.O. Box 425
SF-20101 Turku 10(FI)

(72) Inventor: Koskenniska, Lasse Antero
Liistetie 4 A 2
SF-90650 Oulu 65(FI)

(72) Inventor: Manninen, Kalle Johannes
Emapuuntie 65
SF-90560 Oulu 56(FI)

(74) Representative: Collier, Jeremy Austin Grey et al,
J.A.Kemp & Co. 14, South Square Gray's Inn
London WC1R 5EU(GB)

(54) **Process for the preparation of trimethoprim and novel intermediates for use therein.**

(57) Trimethoprim, i.e. 5-[(3,4,5-trimethoxyphenyl) methyl]-2,4-pyrimidinediamine of the formula

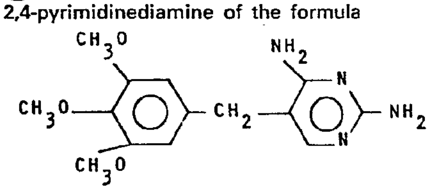

(1)

is advantageously prepared by reacting two moles of 3,4,5-trimethoxybenzaldehyde with 1 mol of 3,3'-(phenylenedi-imino)dipropanenitrile in the presence of an alkaline condensing agent to form a condensation product of formula:

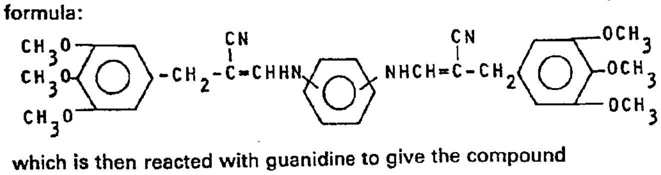

(111)

which is then reacted with guanidine to give the compound of formula (I).

EP 0 067 593 A1

DESCRIPTION

"PROCESS FOR THE PREPARATION OF TRIMETHOPRIM AND NOVEL INTERMEDIATES FOR USE THEREIN"

The present invention provides a novel process for the preparation of trimethoprim, i.e. 5-[(3,4,5-trimethoxyphenyl)methyl]-2,4-pyrimidinediamine of the formula:

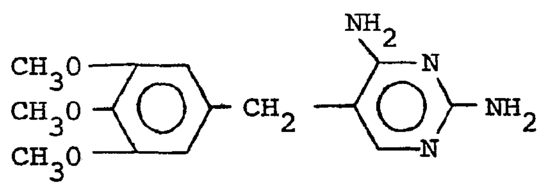

(I)

Trimethoprim is a well-known antimicrobic agent and a sulphonamide synergist.

Many processes are known for the preparation of trimethoprim. The following process is described in British Specification No.957797:

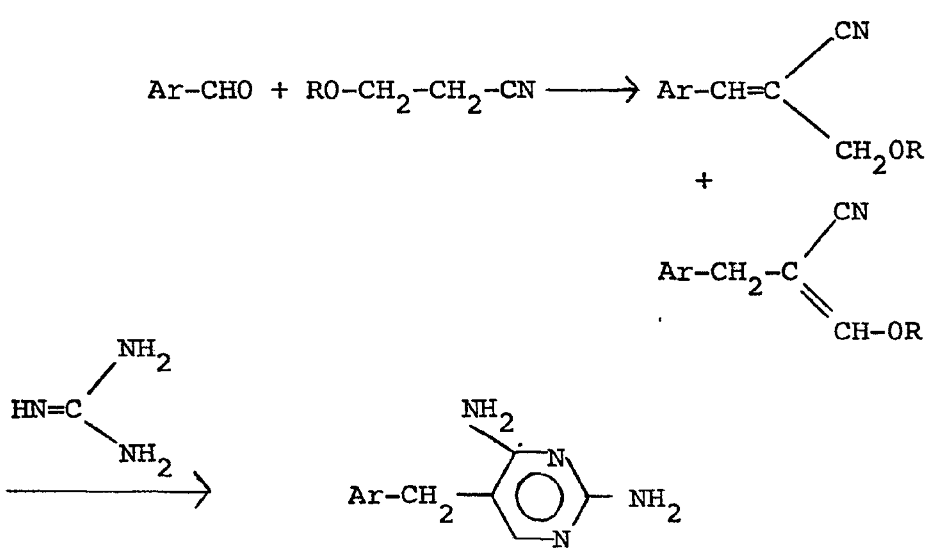

where Ar = 3,4,5-trimethoxyphenyl and R = alkyl.

There are many later modifications of this process. For example British Specification No. 1261455 describes the reaction of an N-substituted β-amino-α-benzyl-propionitrile of the formula

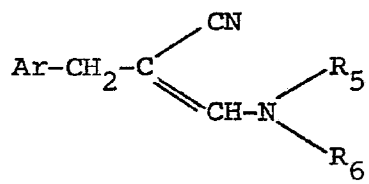 (A)

wherein Ar is for example 3,4,5-trimethoxyphenyl and the $NR_5R_6$ group is an aliphatic, heterocyclic or aromatic amino group, with guanidine to produce trimethoprim. The nitriles of formula (a) are themselves made by the reaction

$$Ar\text{-}CHo + NCCH_2CH_2NR_5R_6 \longrightarrow \quad (A)$$

where AR and $-NR_5R_6$ are as hereinbefore defined. This reaction is effected in the presence of a polar solvent and a base.

The present invention provides a novel, simple process for the preparation of trimethoprim in good yields. According to the invention, 2 moles 3,4,5-trimethoxybenzaldehyde are condensed with 3,3'-(phenylenedi-imino) dipropanenitrile of the formula

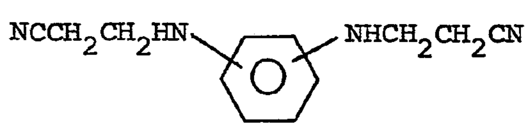 (II)

in the presence of a base, to produce a new intermediate, namely a phenylenedi-imino-N,N'-[α-(3,4,5-trimethoxybenzyl) acrylonitrile] of the formula:

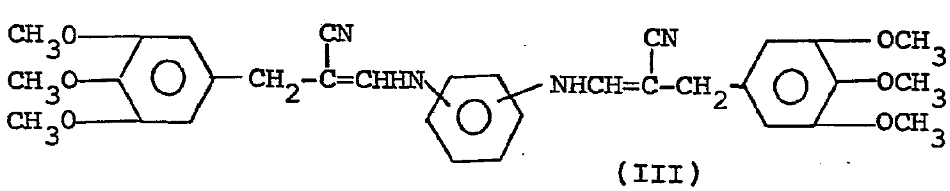

(III)

This intermediate of formula (III) then reacts with 2 moles of guanidine to produce trimethoprim.

The imino groups of the compounds (II) and (III) can be in o-, m- or p-position in relation to each other.

The intermediate of formula (II) surprisingly reacts with 3,4,5-trimethoxybenzaldehyde to give the benzyl intermediate of formula (III) in good yield. Because of steric hindrance in the intermediate (III), it could be assumed that no reaction at all would take place. In the mentioned condensation reaction the benzyl compound of the formula (III) is formed almost entirely, which is advantageous when the intermediate reacts with guanidine. The benzal form, i.e.

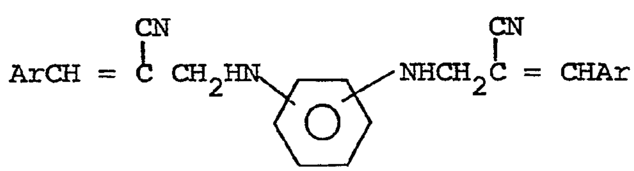

(IV)

wherein Ar = 3,4,5-trimethoxyphenyl, practically does not appear at all.

The condensation of trimethoxybenzaldehyde with the 3,3'-(phenylenedi-imino) dipropanenitrile of formula (II) is carried out in a polar solvent, preferably dimethylsulphoxide, tert. butanol, dimethylformamide, N-methylpyrrolidone, ethylene glycol monomethyl ether, isopropanol or a mixture thereof. Suitable alkaline condensing agents are, e.g., potassium t-butoxide, sodium isopropoxide or sodium methoxide and the temperature of reaction is usually 10 - 30°C, preferably 15 - 20°C. The condensation product obtained may be purified in a very simple manner for instance by evaporating the solvent and adding the residue dropwise to water, which gives the intermediate of formula (III) in crystalline form of high quality.

Trimethoprim may be produced from the intermediate of formula (III) by reaction with guanidine

by refluxing in a suitable solvent, preferably a lower alkanol.

The new process gives a high overall yield of trimethoprim especially when o-phenylenediamine derivatives are used.

The dipropanenitriles of formula (II), which are also novel intermediates, may be made by reaction of acrylonitrile with a phenylenediamine of formula:

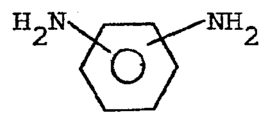

(V)

in the presence of an acid catalyst. The reaction is conveniently effected at an elevated temperature, e.g. 50-100°C., in an aqueous medium in the presence of a catalytic amount of an inorganic acid such as hydrochloric acid and a polymerization inhibitor, e.g. hydroquinone, to stabilize the acrylonitrile.

The invention is illustrated by the following Examples.

Example 1

To a reaction flask containing p-phenylenediamine (65.0 g, 0.6 mol) and 0.7 g hydroquinone, were added 250 ml water followed by acrylonitrile (75 g, 1.42 mol) and, dropwise, 3 ml of concentrated hydrochloric acid. The temperature was raised to 80°C where it was kept for 1.5 hours. The product was precipitated by cooling, filtered off and washed with water. After recrystallization from 200 ml water-ethanol (1:1) 123.3 g (96% of the theoretical) of 3,3'-(p-phenylenedi-imino)-dipropanenitrile was obtained, m.p. 133-136°C.

Example 2

By repeating the process of Example 1 but replacing the p-phenylenediamine with o-phenylenediamine, 118.0 g (92% of the theoretical) of 3,3'-(o-phenylenedi-imino)dipropanenitrile were obtained, m.p. 115-116°C.

Example 3

By repeating the process of Example 1 but replacing the p-phenylenediamine with m-phenylenediamine, 119.5 g (93% of the theoretical) of 3,3'-(m-phenylenedi-imino)dipropanenitrile were obtained, m.p. 167-170°C.

Example 4

3,4,5-Trimethoxybenzaldehyde (4.9 g, 0.025 mol) and 3,3'-(o-phenylenedi-imino)dipropanenitrile prepared as described in Example 2 (2.7 g, 0.0126 mol) were dissolved in a mixture of dimethyl sulphoxide (15 ml) and tert. butanol (20 ml). The solution was cooled to 15°C and potassium t-butoxide (2.8 g, 0.025 mol) was added with stirring in small portions at 15 - 20°C. The stirring was continued overnight. The t-butanol was evaporated, and the residue added dropwise to cold water (150 ml). The light yellow condensation product was separated by filtering, washed with water and dried. Yield 6.7 g (94% of the theoretical) of o-phenylenedi-imino-N,N'-[α-(3,4,5-trimethoxybenzyl)acrylonitrile], m.p. 77 - 80°C.

Example 5

3,4,5-Trimethoxybenzaldehyde (4.9 g, 0.025 mol) was condensed with 3,3'-(p-phenylenedi-imino)-dipropanenitrile prepared as described in Example 1 (2.7 g, 0.0126 mol) in the manner described in Example 4. The product-mixture was added dropwise to water and the reaction product precipitated with a light brown colour. Yield 6.0 g (84% of the theoretical) of p-phenylenedi-imino-N,N'-(3,4,5-trimethoxybenzyl)acrylonitrile], m.p. 162 - 165°C.

Example 6

3,4,5-Trimethoxybenzaldehyde and 3,3'-(m-phenylenedi-imino)-dipropanenitrile prepared as described in Example 3 were condensed together as described in Example 4 and m-phenylenedi-imino-N,N'-[α-(3,4,5-trimethoxybenzyl)acrylonitrile] was obtained in a 73%

yield as a dark brown reaction product, m.p. 76 - 79°C.

Example 7

The condensation product from Example 4 (5.7 g, 0.01 mol) and an alcoholic solution of guanidine (prepared by dissolving 5.7 g, 0.06 mol, of guanidine hydrochloride in 25 ml ethanol and adding 11 ml of 30% methanolic solution of sodium methoxide and filtering off the precipitated sodium chloride) were refluxed with stirring for 4 hours. The solution was allowed to cool and the 5-[(3,4,5-trimethoxyphenyl)methyl]-2,4-pyrimidinediamine which had crystallized out was separated by filtration, and washed with water and cold acetone. The yield was 4.8 g (83% of the theoretical). Melting point 199 - 201°C.

Example 8

The condensation product from Example 5 was reacted with an alcoholic solution of guanidine in a manner described in Example 7. The yield of 5-[(3,4,5-trimethoxyphenyl)methyl]-2,4-pyrimidinediamine was 75% of the theoretical.

Example 9

The condensation product of Example 6 and guanidine were reacted together in the manner described in Example 7 and a 60% yield of 5-[(3,4,5-trimethoxyphenyl)methyl]-2,4-pyrimidinediamine was obtained.

CLAIMS

1. A process for the preparation of 5-[(3,4,5-trimethoxyphenyl)methyl]-2,4-pyrimidinediamine of the formula:

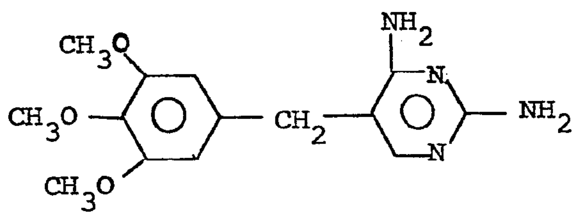

(I)

by reaction of 3,4,5-trimethoxybenzaldehyde with a β-aminoproprionitrile in the presence of a polar solvent and a base, characterised in that the 3,4,5-trimethoxybenzaldehyde is reacted with a 3,3'-(phenylenedi-imino) dipropanenitrile of the formula:

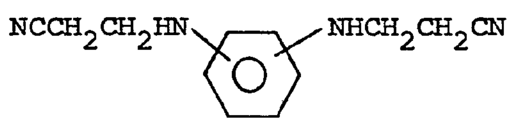

(II)

to produce a condensation product of the formula:

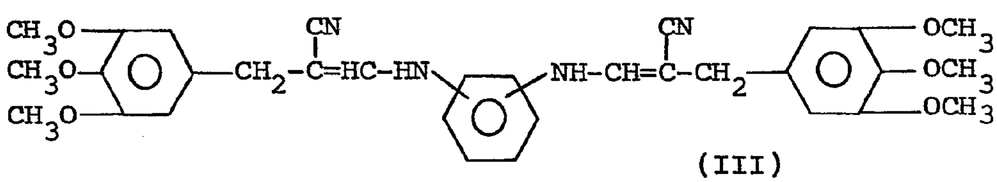

(III)

and the condensation product of formula III is then reacted with guanidine to give a compound of formula I.

2. A process as claimed in claim 1 wherein the said condensation is carried out at a temperature of 10 - 30°C in a polar solvent selected from dimethylsulphoxide, t-butanol, dimethylformamide, N-methylpyrrolidone, ethylene glycol monomethyl ether, isopropanol

or a mixture thereof and in the presence of an alkaline condensing agent selected from potassium t-butoxide, sodium isopropoxide or sodium methoxide.

3. A process as claimed in claim 1 wherein the dipropanenitrile of formula II is prepared by reaction of acrylonitrile with a phenylenediamine of formula:

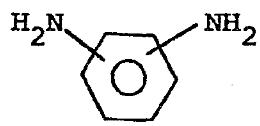

(V)

4. Process for the preparation of a phenylene-di-imino-N,N'-[α-(3,4,5-trimethoxybenzyl)acrylonitrile] of the formula:

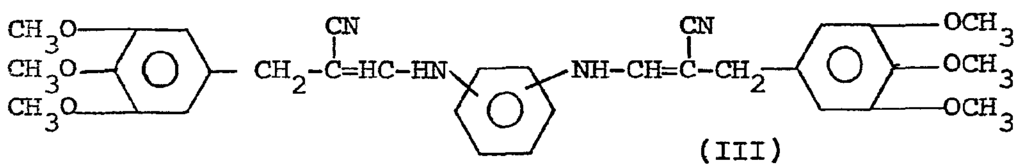

(III)

which comprises reacting 3,4,5-trimethoxybenzaldehyde with a 3,3'-(phenylenedi-imino)propanenitrile of the formula:

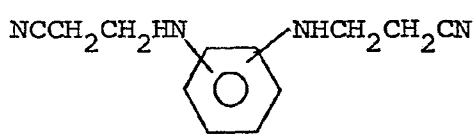

(II)

5. Process for the preparation of a 3,3'-(phenylenedi-imino)propanenitrile of the formula:

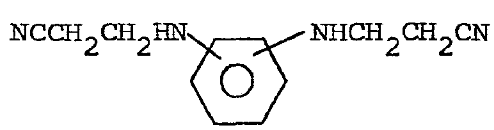

(II)

which comprises reacting acrylonitrile with a phenylene-diamine of the formula:

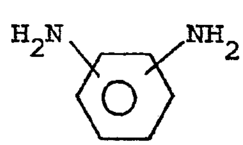

(V)

BAD ORIGINAL

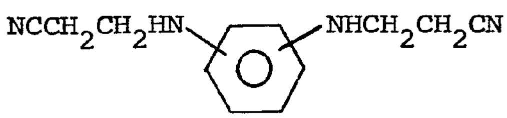 (II)

to produce a condensation product of the formula:

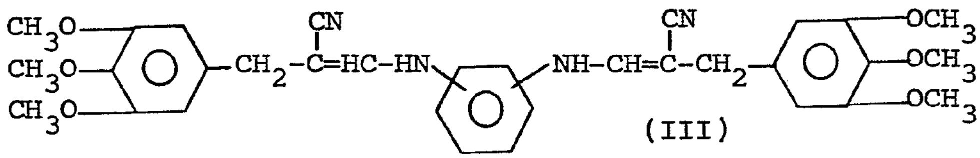 (III)

10. Process for the preparation of a compound as claimed in claim 6 or 7 which comprises reacting acrylonitrile with a phenylene diamine of the formula:

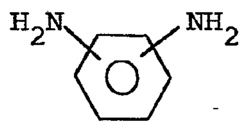 (V)

11. A compound as claimed in claim 6 or 7 for use as an intermediate in the preparation of a phenylenediimino-N,N'-[α-(3,4,5-trimethoxybenzyl)acrylonitrile] as claimed in claim 4 or 5 or of 5-[(3,4,5-trimethoxyphenyl)-methyl]-2,4-pyrimidine diamine.

0067593

European Patent Office

# EUROPEAN SEARCH REPORT

Application number

EP 82 30 2762

| Category | Citation of document with indication, where appropriate, of relevant passages | Relevant to claim | CLASSIFICATION OF THE APPLICATION (Int. Cl. 3) |
|---|---|---|---|
| A | DE-A-2 612 891 (SMITH KLINE) * pages 1-3, 7-14 * --- | 1,2,9 | C 07 D 239/48<br>C 07 C 121/43<br>C 07 C 121/75 |
| D,A | GB-A-1 261 455 (WELLCOME) * pages 1-4, 7-9, 16-22 * --- | 1,2 | |
| X | CHEMICAL ABSTRACTS, vol.76, 1972, page 369, abstract no.25255u, Columbus, Ohio (US) A.N. KOST et al.: "Formation of 1H-2,3-dihydro-2,2,4-trimethyl-1, 5-benzodiazepine from o-phenylenediamine and acetone" & Khim. Geterotsikl. Soedin. 1971, 7(4), 553-5 * the whole abstract * --- | 6,7 | |
| X | GB-A-2 008 598 (ASAHI) * page 6, example 8 * --- | 6 | TECHNICAL FIELDS SEARCHED (Int. Cl. 3) |
| X | CHEMICAL ABSTRACTS, vol.61, 1964, colonne 1794d, Columbus, Ohio (US) O.Y. FEDOTOVA et al.: "Cyanoethylation of diamines II. Cyanoethylation of aromatic diamines" & Zh. Obshch. Khim. 34(1), 181-6 * the whole abstract * ----- | 6,10 | C 07 D 239/00<br>C 07 C 121/00 |

The present search report has been drawn up for all claims

| Place of search | Date of completion of the search | Examiner |
|---|---|---|
| THE HAGUE | 07-09-1982 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82